# EUROPEAN PATENT SPECIFICATION

(11) **EP 0 335 513 B1**
(45) Date of publication and mention of the grant of the patent: **12.05.1993**
(21) Application number: 89302221.0
(22) Date of filing: 06.03.1989
(51) Int. Cl.: A61F 9/00

(54) **Device for assisting in dispensing**
Verabreichungshilfe
Dispositif pour faciliter l'administration

(30) Priority: 31.03.1988 GB 8807728
(43) Date of publication of application: 04.10.1989
(73) Proprietor: Dispomed Limited, Calderstones Liverpool, L18 3LJ (GB)
(72) Inventor: Prebble, Ernest Porter, Calderstones Liverpool L18 3LS (GB)
(74) Representative: Lyons, Andrew John

(56) References cited:
- DE-C- 594 860
- FR-A- 1 263 954
- GB-A- 1 163 903
- US-A- 3 872 866
- US-A- 4 134 403
- US-A- 4 792 334

## Description

This invention concerns a device for assisting dispensing of eye drops from an eye drop container.

Eye drop solution is now mainly supplied in a squeezable plastics container that has its own dispensing nozzle. Dispensing of the eye drop solution is relatively simple for one person applying the drops to the eye of another person but self-dispensing can be extremely difficult especially for those whose grip is not very good such as sufferers from arthritis.

A device for assisting dispensing of eye drops from an eye drop container is known from DE-C-594860 on which the preamble of claim 1 is based.

The object of this invention is to provide a device for assisting dispensing of eye drops from an eye drop container that has its own dispensing nozzle and a removable cap therefor whereby the container is easily handled.

According to the invention there is provided a device for assisting dispensing of eye drops from an eye drop container of flexible plastics material, the device comprising a holder, which permits a nozzle of the container to extend through the holder, means for squeezing the container to emit drops therefrom, the squeezing means comprising two parts relatively movable towards each other to squeeze the container therebetween and guide means whereby the container nozzle can be positioned relative to the eye; characterised in that said guide means is hingedly connected to the holder to be movable to permit removal and replacement of a container cap and that the holder has means for retaining the container against undesired removal.

The squeezing parts may be on limbs that are pivotally connected either at one end in the fashion of nutcrackers. The limbs may be shaped to assist gripping by fingers or may have finger grips attached thereon such as in the form of suitably shaped sleeves.

In a preferred embodiment the guide means is in continuous form, such as an eye bath shape, or cylinder about the nozzle of the eye drop container.

The device of the invention may be moulded from plastics material, for example, by injection moulding of polypropylene.

The invention will now be further described, by way of example only, with reference to the accompanying drawings, in which:-
Figure 1 shows a device for assisting dispensing of eye-drops;
Figure 2 is a partial view from below of the device of Figure 1; and
Figure 3 shows a second device for assisting dispensing of eye-drops.

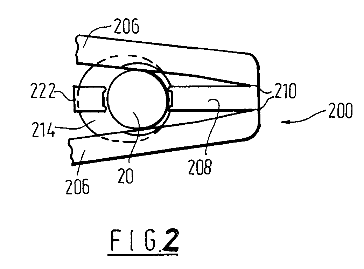

Referring to Figures 1 and 2 of the accompanying drawings, a device 200 for assisting dispensing of eye drops from an eye drop solution container 202 having a dispensing nozzle 204 comprises a pair of handles 206 hingedly attached to either side of a member 208 at one end thereof. The handles 206 and member 208 are preferably moulded in one piece, hinges 210 being of the membrane type. The handles 206 are arranged for relative movement and away from each other. The handles 206 have finger location formations 212 to facilitate gripping of the device by the hand.

The member 208 extends from the hinges between the handles 206 and has at its other end a generally cylindrical part 214 into which the eye drop container 202 fits, leaving a substantial portion of the container exposed below the part 214. It is that part of the container that can be squeezed by pulling together the handles 206 of the device 200. It has been found that only slight pressure on the container by the handles is required to discharge a drop of eye solution from the nozzle 204 of the container 202.

To facilitate positioning of the device 200 relative to the eye, the handles 206 are bent at 218. In addition a cylindrical guide means 220 is hingedly connected to the member 208 at 222. The guide means 220 is intended to be placed over the eye and is sized so as to hold the eye drop container nozzle at a suitable distance from the eye. The guide means 220 may also serve to hold the eyelids open slightly due to the pressure thereon and, if of opaque material, may cut out sufficient light from the eye to prevent blinking which might otherwise hinder administration of the eye solution.

Finally, the arms 206 are provided near their free ends with serrated indentations 230 between which a cap of an eye solution container can be gripped to facilitate removal thereof from the container.

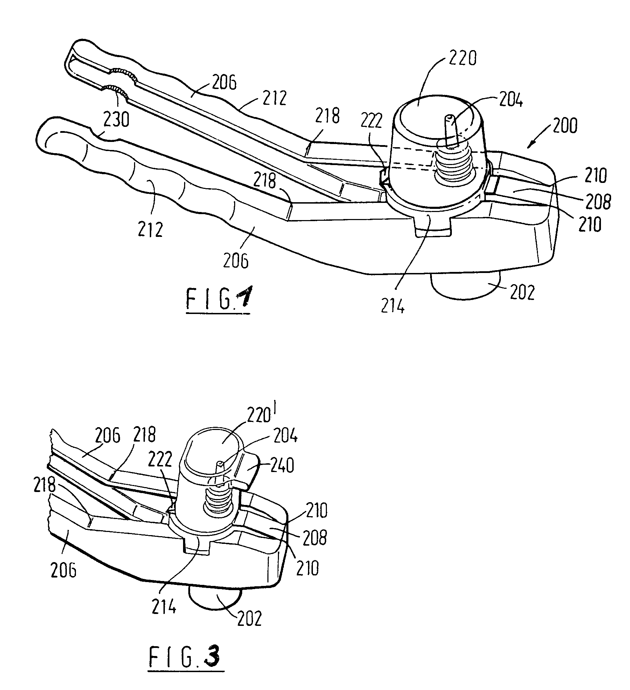

In Figure 3 is illustrated a modification to the device of Figure 1. Thus, in Figure 3 the guide means 220' has a protrusion 240 on its edge towards the hinge end of the device. The protusion 240 is intended to abut against the bone above the eye socket so that undue pressure is not exerted on, for example, cataract scars by the guide means.

All of the illustrated embodiments may be produced from plastics material, such as polypropylene say by injection moulding.

## Claims

1. A device for assisting dispensing of eye drops from an eye drop container of flexible plastics material, the device (200) comprising a holder (214), which permits a nozzle (204) of the container (202) to extend through the holder, means (206) for squeezing the container to emit drops therefrom, the squeezing means comprising two parts relatively movable towards each other to squeeze the container therebetween and guide means (220) whereby the container nozzle can be positioned relative to the eye; characterised in that said guide means (220) is hingedly connected (222) to the holder to be movable to permit removal and replacement of a container cap and that the holder has means for retaining the container against undesired removal.

2. A device as claimed in claim 1, wherein the means (26, 214) for holding the container is generally tubular.

3. A device as claimed in any one of claims 1 or 2, wherein said squeezing means are limbs (206) pivotally connected at one end.

4. A device as claimed in any one of claims 1 to 3, wherein said guide means (220) is generally cylindrical and surrounds the container nozzle.

## Patentansprüche

1. Ein Gerät zur Unterstützung der Abgabe von Augentropfen aus einem Augentropfenbehälter aus biegsamen Kunststoffmaterial, wobei das Gerät (200) einen Halter (214) aufweist, durch den sich eine Tülle des Behälters (202) erstreckt, eine Vorrichtung, mit deren Hilfe Tropfen aus dem Behälter herausgedrückt werden können, wobei die Vorrichtung zum Zusammendrücken zwei Teile aufweist, die relativ aufeinander zu bewegt werden können, um den Behälter dazwischen zusammendrücken zu können, und eine Führungsvorrichtung (220), mit deren Hilfe die Behältertülle relativ zum Auge positioniert werden kann,
dadurch **gekennzeichnet,**
daß die Führungsvorrichtung (220) scharniermäßig (222) mit dem Halter verbunden ist, um zum Entfernen und Ersetzen einer Behälterkappe beweglich zu sein, und daß der Halter eine Einrichtung zum Festhalten des Behälters gegen unerwünschtes Entfernen aufweist.

2. Ein Gerät nach Anspruch 1,
dadurch **gekennzeichnet,**
daß die Vorrichtung zum Halten des Behälters im allgemeinen rohrförmig ist.

3. Ein Gerät nach Anspruch 1 oder 2,
dadurch **gekennzeichnet,**
daß die Vorrichtung zum Zusammendrücken zwei Griffe (206) sind, die an einem Ende gelenkig miteinander verbunden sind.

4. Ein Gerät nach einem der Ansprüche 1 bis 3,
dadurch **gekennzeichnet,**
daß die Führungsvorrichtung (220) im allgemeinen zylindrisch ist und die Behältertülle umgibt.

## Revendications

1. Dispositif pour aider à la distribution de gouttes occulaires à partir d'un flacon de gouttes occulaires en matériau plastique flexible, le dispositif (200) comprenant un support (214) qui permette à une canule (204) du conteneur (202) de traverser le support, un moyen (206) pour comprimer le conteneur pour en faire sortir des gouttes, le moyen de compression comprenant deux parties mobiles l'une par rapport à l'autre pour comprimer le conteneur entre elles et des moyens de guidage (220) par lesquels la canule du conteneur peut être positionnée par rapport à l'oeil, caractérisé en ce que ledit moyen de guidage (220) est relié de manière articulée (222) au support de manière à être mobile pour permettre l'enlèvement et le remplacement d'une tête de conteneur et en ce que la support possède des moyens pour retenir le conteneur à l'encontre d'un retrait non désiré.

2. Dispositif selon la revendication 1, dans lequel le moyen (26,214) pour maintenir le conteneur est de forme générale tubulaire.

3. Dispositif selon l'une quelconque des revendications 1 et 2, dans lequel les moyens de compression sont des branches (206) montées pivotantes à une extrémité.

4. Dispositif selon l'une quelconque des revendications 1 a 3, caractérisé en ce que lesdits moyens de guidage (220) sont de forme générale cylindrique et entourent la canule du conteneur.
